# EUROPEAN PATENT SPECIFICATION

(11) **EP 2 611 371 B1**
(45) Date of publication and mention of the grant of the patent: **05.04.2017**
(21) Application number: 11821200.0
(22) Date of filing: 28.08.2011
(51) Int. Cl.: A61B 17/56, A61B 17/72, A61B 17/68, A61B 17/84

(54) **ORTHOPEDIC IMPLANT FOR TREATMENT OF BONE DEFORMITIES**
ORTHOPÄDISCHES IMPLANTAT ZUR BEHANDLUNG VON KNOCHENVERFORMUNGEN
IMPLANT ORTHOPÉDIQUE DESTINÉ AU TRAITEMENT DE DÉFORMATIONS OSSEUSES

(30) Priority: 29.08.2010 US 377952 P
(43) Date of publication of application: 10.07.2013
(73) Proprietor: Bonfix Ltd., 91391 Jerusalem (IL)
(72) Inventor: HERZOG, Rafi, 30992 Bat Shlomo (IL); BARKAI, Nir, 44221 Kfar Saba (IL); SHAHAR, Mark, 58459 Holon (IL); ROBINSON, Dror, 99788 Kfar Shmuel (IL); SCHON, Lew, Pikesville Maryland 21208-3312 (US)
(74) Representative: Pearl Cohen Zedek Latzer Baratz UK LLP
(86) International application number: PCT/IB2011/053763
(87) International publication number: WO 2012/029008

(56) References cited:
- BE-A6- 1 010 569
- US-A- 5 433 665
- US-A- 5 507 812
- US-A- 5 941 885
- US-A- 5 941 885
- US-A1- 2005 240 188
- US-A1- 2005 240 188
- US-A1- 2007 161 991
- US-A1- 2008 208 252
- US-A1- 2008 275 563
- US-A1- 2008 288 070
- US-A1- 2009 287 246
- US-A1- 2009 287 246
- US-A1- 2010 076 504
- US-A1- 2010 211 071

## Description

### FIELD OF THE INVENTION

The present invention relates generally to devices for orthopedic surgical procedures, and specifically to implantable devices for treatment of deformities of the bones in the foot.

### BACKGROUND OF THE INVENTION

"Hallux valgus" or "hallux abducto valgus" is a common disorder of the forefoot, which is associated with bunion deformity. The term refers to an abnormal slant of the big toe relative to the second toe. It is one of the most common pathologic conditions affecting the foot and toes.

Hallux valgus results from a medial deviation of the first metatarsal bone and lateral deviation and/or rotation of the big toe (hallux), with or without medial soft-tissue enlargement of the first metatarsal head (bunion). In normal feet, the angle between the first and second metatarsal bones (known as the Inter-Metatarsal Angle, or IMA) is typically in the range of 6-90. In hallux valgus, this angle may increase to more than 12° in moderate cases and more than 16° in severe cases. Treatment of hallux valgus often includes surgical intervention to reduce the inter-metatarsal angle.

A number of surgical devices and techniques have been developed for reduction of the inter-metatarsal angle. For example, U.S. Patent Application Publication 2010/0152752 describes a method and apparatus for bunion repair using a suture-passing K-wire. (A K-wire, or Kirschner wire, is a sharpened, smooth metal pin, widely used in orthopedic surgery, which is driven into the bone using a drill.) The K-wire is used to pass a suture through the first and second metatarsal bones. The first and second metatarsals are pushed together to correct the inter-metatarsal angle deformity, and the suture is tied in this position to hold anchor buttons against the bones. Arthrex, Inc. (Naples, Florida) offers a commercial product of this sort, known as the Mini TightRope, which is also described in U.S. Patent 7,875,058.

PCT International Publication WO 2009/018527 describes a fixation and alignment device for use in orthopedic surgery for the correction of bone deformities. The device is part of an anchoring system that is said to be suitable for surgical repair of hallux valgus and other conditions. The system is used to anchor two or more sections of bone or other body parts and to align one section relative to another.

PCT International Publication WO 2010/093696 describes devices for treating hallux valgus using dynamic tensioning components or heat shrinkable components to urge two metatarsals together to treat a bone deformity. The dynamic tensioning component exhibits elasticity and has a tensioned state and an untensioned state. In the tensioned state, the component urges first and second anchors, attached to the first and second metatarsal bones, toward each other.

### SUMMARY

Embodiments of the present invention provide implantable surgical devices for treatment of bone deformities, such as hallux valgus.

There is therefore provided, in accordance with an embodiment of the present invention, an implantable device, comprising a first cylindrical anchor, configured to be implanted inside a first bone, and a second cylindrical anchor, configured to be implanted inside a second bone, adjacent to the first bone. A cord extends between the first and second anchors. A shock absorber, contained within at least one of the first and second anchors, is coupled to the cord so as to deform in response to a force exerted on the cord. The shock absorber includes a spring and a force adjuster, which is configured to adjust a baseline deformation of the spring, by compressing the spring. The force adjuster includes a first screw contained within said at least one of the first and second anchors. The device is characterized with respect to the implantable device disclosed in BE-A-1010569 in that the first and second cylindrical anchors have respective first and second diameters, the first diameter being greater than the second diameter.

The device may further comprise an adjustment mechanism including a second screw coaxial with the first screw to adjust a length of the cord extending between the first and second anchors.

An adjustment tool, which includes first and second coaxial drivers, may be used to engage and turn the first and second screws, respectively. In a disclosed embodiment, the adjustment tool includes a tip, which engages a socket in the at least one of the first and second anchors so as to prevent rotation of the cord as the first and second screws are turned. The tip may be configured to be broken off the tool after adjustment of the force adjuster.

In one embodiment, the shock absorber includes first and second shock absorbing elements, having different, respective responses to the force exerted on the cord.

The first and second anchors may include respective collars at respective proximal ends of the anchors for engaging respective surfaces of the first and second bones, wherein the device includes a fastener configured to engage a distal end of the second anchor so as to secure the second anchor in the second bone. Additionally or alternatively the collar of the first anchor may contain one or more holes that are configured to receive a suture for attachment to tissue in proximity to the first bone.

Typically, the cord includes multiple strands of a metal wire, and the first and second anchors are sized and shaped for implantation inside the first and second metatarsal bones, respectively.

A device provided in accordance with an embodiment of the present invention may be used in a method of surgical treatment, which method includes implanting a first anchor inside a first metatarsal bone of a foot, and implanting a second anchor, which is connected to the first anchor by a cord, inside a second metatarsal bone of the foot. A shock absorber contained within one of the first and second anchors is coupled to the cord so as to deform in response to a force exerted on the cord.

A baseline deformation of the shock absorber may be adjusted so as to alter the response to the force.

It is disclosed that the method may include, after implanting the first and second anchors, adjusting a length of the cord that extends between the first and second anchors using an adjustment mechanism inside at least one of the anchors so as to modify an inter-metatarsal angle of the foot.

The first and second anchors are cylindrical and have respective first and second anchor diameters, wherein the first anchor diameter is greater than the second anchor diameter, and implanting the first and second anchors includes drilling first and second bores respectively in the first and second bones, the bores having respective first and second bore diameters in accordance with the first and second anchor diameters, passing the second anchor through the first bore into the second bore, and inserting the first anchor into the first bore.

In a disclosed embodiment, passing the second anchor includes passing a K wire through the first and second bores, pulling a tube, which is attached to a distal end of the second anchor, over the K wire in a distal direction through the first and second bores until the second anchor is inserted into the second bore. The tube may be turned while pulling in order to screw the second anchor into the second bone.

Alternatively or additionally, implanting the second anchor may include attaching a fastener to a distal end of the second anchor protruding from the bone so as to secure the second anchor in the bone.

The present invention will be more fully understood from the following detailed description of the embodiments thereof, taken together with the drawings in which:

### BRIEF DESCRIPTION OF THE DRAWINGS

Fig. 1 is a schematic top view of the bones of a foot, in which a device for reducing inter-metatarsal angle has been implanted in accordance with an embodiment of the present invention;
Fig. 2A is a schematic, pictorial illustration of an implantable device, in accordance with an embodiment of the present invention;
Fig. 2B is a schematic, pictorial illustration of an anchor used in an implantable device, in accordance with an alternative embodiment of the present invention;
Fig. 3 is a schematic, sectional view of the device of Fig. 2A;
Fig. 4 is a schematic, pictorial illustration showing details of the proximal end of an implantable device, in accordance with an embodiment of the present invention;
Fig. 5 is a schematic, pictorial illustration showing the distal end of a surgical adjustment tool, in accordance with an embodiment of the present invention;
Fig. 6 is a schematic, sectional view of a surgical device that has been implanted in the first and second metatarsal bones, in accordance with an alternative embodiment of the present invention;
Fig. 7 is a schematic top view of a foot showing a preparatory stage in a procedure for implantation of a device of the present invention in the first and second metatarsal bones; and
Figs. 8-11 are schematic, pictorial views of the first and second metatarsal bones showing successive stages of the procedure begun in Fig. 7; and
Fig. 12 is a schematic, sectional view showing the final stage in the procedure of Figs. 7-11.

### DETAILED DESCRIPTION OF EMBODIMENTS

### OVERVIEW

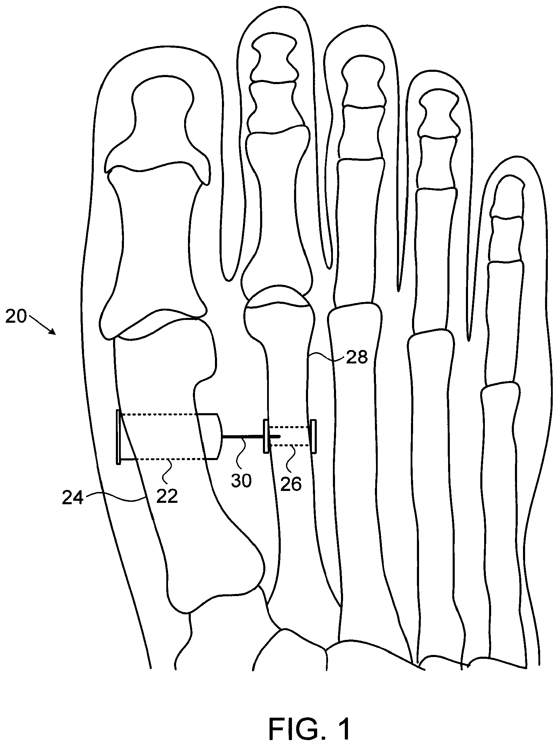

Fig. 1 is a schematic top view of the bones of a foot, in which a device 20 for reducing the inter-metatarsal angle of the present invention has been implanted. Device 20 comprises a first anchor 22, which is implanted in a first metatarsal bone 24, and a second anchor 26, implanted in a second metatarsal bone 28. The anchors are connected by a flexible cord 30, which extends between the anchors (and thus between the two metatarsal bones). An adjustment mechanism, which is described in detail hereinbelow, enables the surgeon, after implantation of the anchors in the bones, to adjust the length of the cord extending between the first and second anchors. The surgeon can thus modify the inter-metatarsal distance, i.e. the IMA, meaning the angle between the respective axes of bones 24 and 28.

In the description that follows, it is assumed, for clarity of explanation, that the "first anchor" is implanted in the first metatarsal, while the "second anchor" is implanted in the second metatarsal. In alternative embodiments, however, certain of the functions and features of the two anchors may be exchanged. Therefore, in the present patent application and in the claims, it should be understood that the terms "first" and "second" are used arbitrarily in relation to the anchors and do not signify which anchor is to be implanted in which of the bones unless the specific context in which the terms are used indicates otherwise.

Although the devices and methods described herein relate specifically to the metatarsal bones and correction of conditions such as hallux valgus, the principles of the present invention may similarly be applied in other orthopedic applications, and particularly in treatments to modify the spacing between adjacent bones. For example, the devices described hereinbelow may be modified for implantation in the metacarpal bones. Other examples of possible applications include closing of sternal osteotomies after open heart surgery; repair of rib fractures, allowing immediate immobilization of multiply fractured ribs; reduction of acromioclavicular dislocations; reduction of ulnar or fibular dislocated metacarpophalangeal or metatarsophalangeal joints, including treatment of Lisfranc fracture dislocation; treatment of ankle syndesmosis injury; dynamic linking of adjacent vertebrae, as an alternative to spinal fusion; treatment of flat foot; and treatment of hammer toe.

### IMPLANTABLE DEVICES

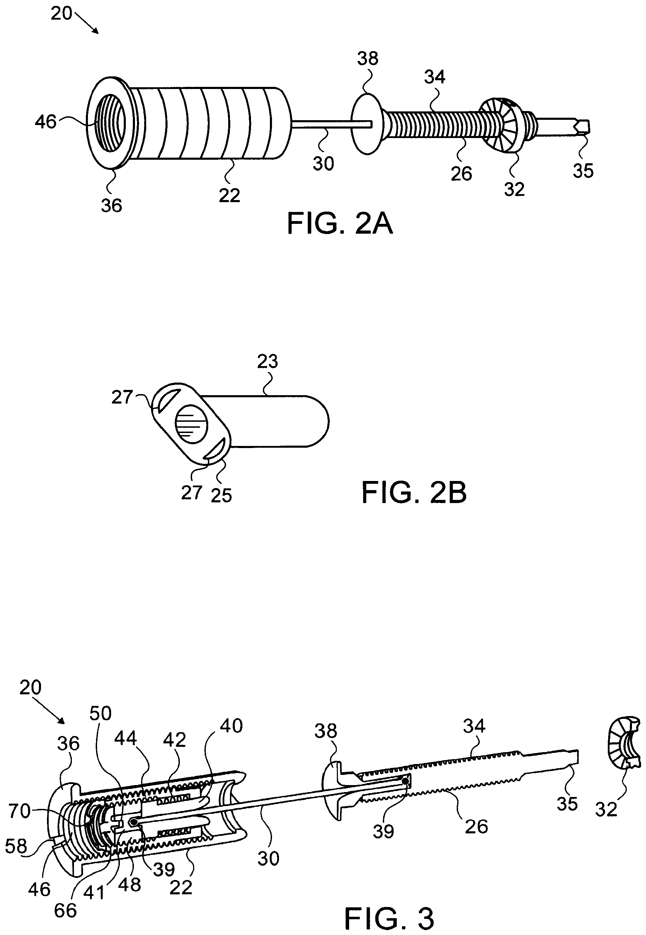

Figs. 2A and 3 schematically show details of device 20, in accordance with an embodiment of the present invention. Fig. 2A is a pictorial, external view of the parts of the device, while Fig. 3 is a sectional view. Anchors 22 and 26 are made from a rigid biocompatible material, such as 316LVM-type stainless steel or titanium alloy, with a cylindrical shape for insertion into cylindrical bores that are drilled in the bones. The diameter of anchor 22 is greater than that of anchor 26, for reasons that will be explained below. For example, anchor 22 may be 12 mm long and 6 mm in diameter, while anchor 26 is 11 mm long and 3 mm in diameter. Alternatively, other dimensions may be chosen depending, *inter alia,* on the dimensions and condition of the bones in which the anchors are to be implanted. Anchors 22 and 26 may be coated with a bone growth promoter, such as hydroxyapatite.

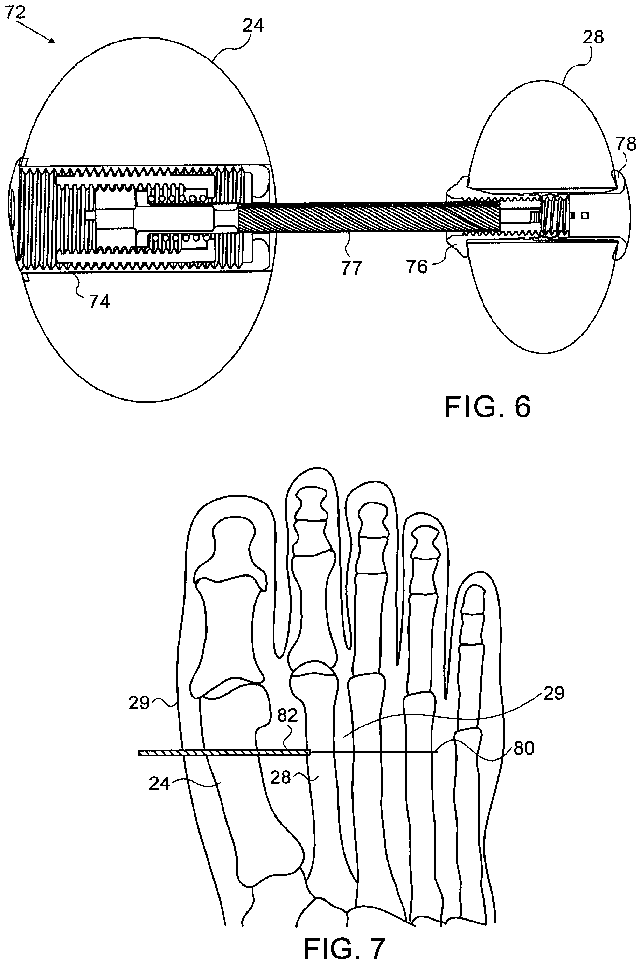

Anchor 26 in this embodiment has an external thread 34, which accepts a nut 32 over a distal end 35 of the anchor (i.e., the end that is inserted through the bone) and may also be used to screw anchor 26 into the bone itself. Anchors 22 and 26 have respective collars 36, 38 at their proximal ends, which engage the bone surface when the anchors have been completely inserted into the respective bores. Nut 32 is then fastened onto thread 34 so as to secure anchor 26 firmly in place in the bone. (Alternatively, a snap-on connection may be used, as shown below in Fig. 6.)

Cord 30 may comprise any flexible (though inelastic), biocompatible material of sufficient strength to withstand the forces exerted by and on the bones of the foot. The cord may comprise either a single strand or multiple strands of a suitable polymer or metal filament. For example, cord 30 may comprise a braided cable made from 316LVM-type stainless steel wire, with an overall diameter of about 0.5 mm. Cord 30 is held inside anchor 22 by a holder 40, with a head 41 attached to its proximal end. Cord 30 may be retained inside holder and collar 38, for example, by means of knots 39 tied at the ends of the cord, or by welding, or by any other suitable means of fastening. In an alternative embodiment (not shown in the figures but considered to be within the scope of the claims), anchors 22 and 26 may be connected by multiple cords, which are configured as a sort of "hammock" between the bones, in order to avoid and relieve local stress points.

Holder 40 also retains and compresses a spring 42. A screw 48 controls the position of holder 40 and thus adjusts the degree of compression and the baseline force on spring 42. Screw 48 has an outer thread, which travels along an inner thread in a wider screw 44. Screw 44 has an outer thread, which travels along a matching internal thread 46 in anchor 22.

Spring 42 controls the tension in cord 30. The spring typically comprises an elastic biocompatible material, such as stainless steel. Alternatively, device 20 may comprise other sorts of mechanically-loaded elements that create mechanical resistance while deforming, such as a flexible polymer or viscoelastic material, a magnetic element applying mechanical force during movement, or a pneumatic or hydraulic element configured to resist geometrical movement.

The compression of spring 42 can be adjusted by rotation of screw 48 (as described in greater detail hereinbelow). Turning screw 48 clockwise (assuming thread 48 to be right-handed) compresses the spring, creating a corresponding "zero-state tension" in cord 30. For example, the zero-state tension may be set to a value in the range between approximately 10 and 15 Newton, although higher and lower values of tension may also be used, depending on clinical conditions.

The longitudinal position of holder 40 within anchor 22 is controlled by an adjustment mechanism comprising screw 44, which travels along internal thread 46 inside the anchor. For example, turning screw 44 counterclockwise (assuming thread 46 to be right-handed) causes holder 40 to shift longitudinally in the proximal direction, thus drawing cord 30 into anchor 22. In this manner, the length of the cord extending between the anchors is reduced, and the inter-metatarsal angle is reduced accordingly. Alternatively, screw 44 may be turned clockwise to play out the cord and thus reduce the force exerted between the metatarsal bones.

The adjustment mechanism in device 20 enables the surgeon to precisely, easily and smoothly control the inter-metatarsal angle after implantation of the anchors. Adjustment may be achieved simply by turning screw 48, rather than having to manually attempt to tie a suture at the appropriate length as in methods that are known in the art. Furthermore, the configuration of device 20, with screw 48 accessible at the proximal end of anchor 20, makes it possible for the surgeon to readjust the length of cord 30 at a later time if desired. Although the pictured embodiments specifically show a screw-based adjustment mechanism, and this mechanism is advantageous both for initial implantation and subsequent adjustment, other types of mechanisms, such as clips or snaps, may alternatively be used.

Spring 42 serves as a shock absorber, by deforming in response to forces exerted on cord 30. Typically, in the course of walking, metatarsal bones 24 and 28 tend to move cyclically apart and back together, thus exerting a cyclical force on cord 30. In addition, stronger forces may be exerted on the cord when the foot receives a sudden impact, due to jumping or kicking, for example. These forces can fatigue both cord 30 and the bones around anchors 22 and 26, and may lead eventually to failure of device 20 or, what is worse, fractures of the bones. Spring 42 absorbs a part of these forces and thus helps to protect against device failure and bone fracture.

As noted above, the surgeon can adjust the initial tension in spring 42 by means of a force adjuster, in the form of screw 48. Turning this screw causes it to travel longitudinally along an internal thread within screw 44, with which screw 48 is coaxial. This longitudinal motion of screw 48 presses or releases cord holder 40 against spring 42, thus increasing or decreasing the baseline compression of the spring and altering its response to force exerted on cord 30.

In an alternative embodiment (not shown in the figures), the shock absorber in device 20 may comprise two (or more) shock absorbing elements with different responses to the force exerted on cord 30. For example, spring 42 may comprise two parts, in series or in parallel. A first part with a low spring constant absorbs small forces over a certain initial range of movement. Once this range is exceeded, the second part of the spring, with a higher spring constant, absorbs the excess force. This sort of combined shock absorber can enhance patient comfort and reduce the chances of damage to the device and to the bones in which the device is implanted.

Fig. 2B is a schematic, pictorial illustration of an anchor 23 for use in an implantable device, in accordance with an alternative embodiment of the present invention. Anchor 23 is functionally similar to anchor 22 and may be used in device 20, for example, in place of anchor 22. Anchor 23 comprises a collar 25, which engages the surface bone 24 when the anchor is implanted inside the bone. Collar 25 contains one or more holes 27 through which a suture (not shown) can be passed for attachment to tissue in proximity to the bone. This suture may be passed through the capsule over the bunion, thus allowing proximal pull of the capsule and 20 anchoring it to device 20. To facilitate passage of the suture needle through holes 27, they are located relatively close to the rim of the collar in a diagonal orientation, so as to allow slippage of the needle between the rim of the collar and the bone.

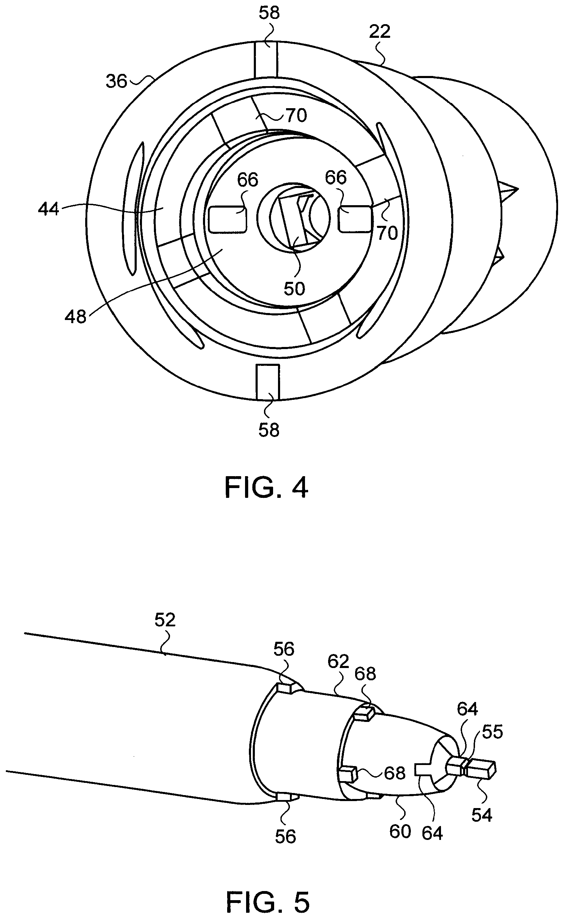

Reference is now made to Figs. 4 and 5, as well as to Fig. 3, which show details of how the cord adjustment mechanism and shock absorber in anchor 22 are adjusted, in accordance with an embodiment of the present invention. Fig. 4 is a schematic, pictorial end view of anchor 22 (showing the proximal end of the anchor, which appears at the left side in Figs. 2 and 3). Fig. 5 is a schematic, pictorial illustration showing the distal end of a surgical adjustment tool 52 that can be used conveniently to make adjustments after anchors 22 and 26 have been implanted in bones 24 and 28.

To begin the adjustment procedure, a fixed tip 54 of tool 52 is inserted into a central socket 50 of head 41 inside anchor 22. Head 41 is connected to cord holder 40, as noted above. In the pictured embodiment, socket 50 and tip 54 are both square, but other matching shapes may similarly be used. The purpose of socket 50 and tip 54 is to prevent rotation of cord 30 as screws 44 and 48 are turned. Similarly, protrusions 56 at the periphery of tool 52 are fixed and engage recesses 58 in collar 36 to prevent rotation of anchor 22 after it has been implanted in bone 24.

Screws 48 and 44 can be adjusted separately and independently by turning drivers 60 and 62, respectively. The drivers, which are coaxial, are typically controlled by respective knobs or wheels at the proximal end of tool 52 (not shown). Recesses 64 on driver 60 engage protrusions 66 on screw 48, while protrusions 68 on driver 62 engage recesses 70 on screw 44. Thus, in a typical procedure, the surgeon holds tool 52 and tip 54 still, without rotation, while rotating each of drivers 60 and 62 in turn in order to achieve the desired baseline compression of spring 42 and zero-state tension on cord 30, along with the desired length of cord 30 between anchors 22 and 26.

In an alternative embodiment, tool 52 is provided as part of a surgical kit, attached to implant 20. In this embodiment, tip 54 may be attached to socket 50 by various means, such as by laser welding. In order to detach tool 52 from implant 20 after implantation, tip 54 may be weakened, by a groove 55 formed near the proximal end of the tip, for example, to enable the operator to easily disengage tool 52 by breaking off tip 54 at the conclusion of the procedure.

Although tool 52 is shown in the pictured embodiments as a single comprehensive tool, which performs all the functions described above, these functions may alternatively be performed by two or more different tools, which are configured to perform the various adjustment functions separately.

Fig. 6 is a schematic, sectional view of a surgical device 72 that has been implanted in first and second metatarsal bones 24, 28. Device 72, like device 20 described above, comprises anchors 74 and 76, which are respectively implanted into bones 24 and 28 and are connected by a cord 77. Device 72 differs from device 20 in two main respects:
- Anchor 76 is secured in place within the bone by a snap-on fastener 78, which is attached to the distal end of anchor 76 after the anchor has been inserted through bone 28.
- An internal thread within anchor 76 enables cord 77 to be screwed in and out of the anchor, thereby serving as an additional part of the cord length adjustment mechanism.

### USES OF THE IMPLANTABLE DEVICES

Fig. 7 is a schematic top view of a foot showing a preparatory stage in a procedure for treatment of hallux valgus using device 20. As an initial step, the surgeon may make incisions at locations 29, i.e., at the medial end of the first metatarsal and the lateral end of the second metatarsal. The surgeon may then remove the medial eminence (excess tissue) from the bunion on first metatarsal bone 24 and may cut the adductor tendon away from the first metatarsal bone so that the first and second metatarsal bones can be brought closer together. These surgical steps are known in the art and are outside the scope of the present patent application.

The surgeon first reduces the distance between first and second metatarsal bones 24, 28 by applying force on the first metatarsal toward the second metatarsal, either by hand or using a suitable tool, such as a special-purpose clamp (not shown). Following this step, the surgeon, using a drill guide (not shown), drills a K-wire 80 percutaneously through first and second metatarsal bones 24, 28. The K-Wire diameter may be, for example, 1.1 mm. The surgeon then drills through bones 24 and 28 using a cannulated drill 82 over K-wire 80. In the present example, drill 82 has a diameter of 2.8 mm, slightly smaller than the outer diameter of anchor 26.

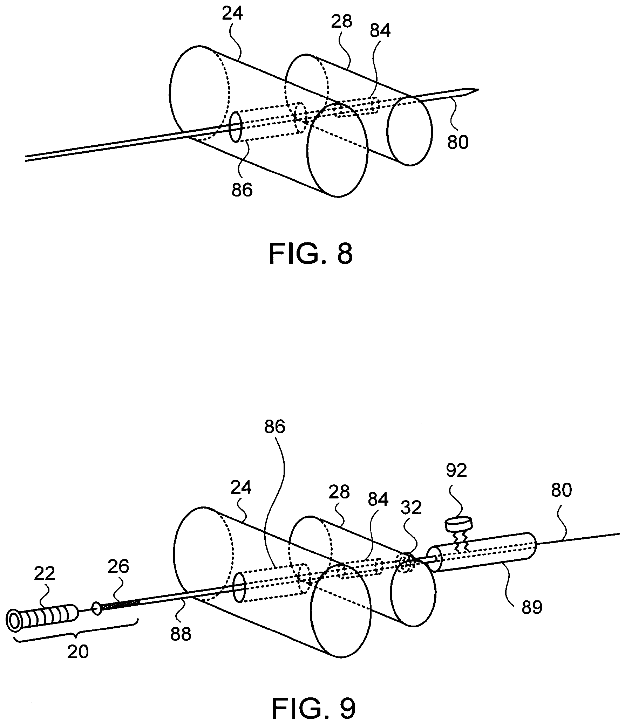

Figs. 8-11 are schematic, pictorial representations of bones 24 and 28, showing successive stages in the surgical procedure. In Fig. 8, a 2.8 mm bore 84 has been drilled in second metatarsal bone 28. The surgeon then uses a larger drill, for example, 6 mm in diameter, to create a larger bore 86 in first metatarsal bone 24. K-wire 80 remains in the bores at this stage.

To aid in insertion of anchors 22 and 26, a tube 88, such as a plastic pipe or cannulated metal tube (made from 316-type stainless steel, for example), is used as an surgical aid. Tube 88 may have a handle 89 attached to facilitate manipulation by the surgeon, as well as a quick-connect mechanism 91 for connecting to distal end 35 of implant 26. Mechanism 91 may comprise, for example, an internal screw thread in tube 88, which mates with an external screw thread on distal end 35.

As shown in Fig. 9, the surgeon slides tube 88 over K-wire 80 and thus through bores 84 and 86 and then attaches the distal end of tube 88 to anchor 26 using mechanism 91. Next, the surgeon pulls the K-wire out of the bores in the distal direction (i.e., to the right in the view shown in Fig. 7). The surgeon then pulls the tube in the distal direction to draw anchors 26 and 22 through bore 86 in turn, until the anchors are inserted fully into bores 84 and 86, respectively. The surgeon may turn tube 88 while pulling in order to screw anchor 26 into bore 84.

Alternatively, tube 88 may be pre-attached to implant 26 at the factory by laser welding, for example. In this case, handle 89 may be pulled over the distal end tube 88 from bore 86 to 84 and secured to the tube by a screw knob 92. In other respects, the procedure is carried out as described above.

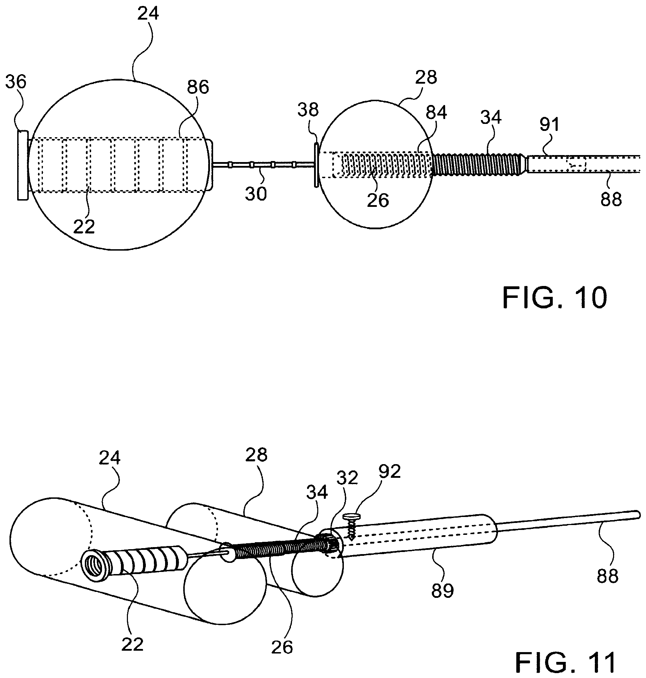

The final position of anchors 26 and 22 in bores 84 and 86 is shown in Fig. 10. Collars 36 and 38 engage the respective surfaces of bones 24 and 28 on the proximal side, while thread 34 and tube 88 protrude outward from bore 84 on the distal side. To secure anchor 26 in place, the surgeon loosens tool 89 from tube 88 using knob 92 and slides nut 32, which resides on tool 89, over tube 88, as shown in Fig. 11. The surgeon then tightens the nut over thread 34 using tool 89 to engage the distal side of bone 28.

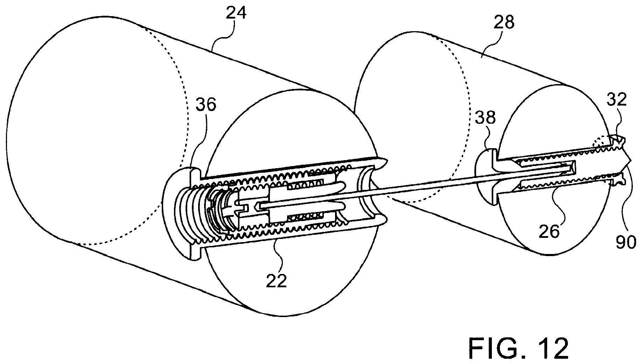

Fig. 12 is a schematic, sectional view showing the final stage in the procedure. The surgeon cuts off the excess of thread 34 (along with the connected tube 88) that protrudes distally out of second metatarsal bone 28, using a suitable cutting tool. Anchor 26 is left with a shortened end 90 protruding slightly out of the bone on the distal side. The surgeon uses tool 52, as described above (Figs. 4 and 5), to adjust the cord length and spring baseline deformation. The surgeon then closes the incisions, completing the procedure. The surgeon may use holes 27 to pass a suture for attachment to tissue in proximity to the bone, thus allowing proximal pull of the capsule and anchoring it to device 20.

Although Figs. 7-12 illustrate a particular procedure for implantation and adjustment of device 20, alternative surgical methods may also be used.

## Claims

1. An implantable device (20), comprising:
a. a first cylindrical anchor (22), configured to be implanted inside a first bone (24);
b. a second cylindrical anchor (26), configured to be implanted inside a second bone (28), adjacent to the first bone;
c. a cord (30) extending between the first and second anchors; and
d. a shock absorber contained within at least one of the first and second anchors and coupled to the cord so as to deform in response to a force exerted on the cord;
said shock absorber comprising a spring (42) and a force adjuster, which is configured to compress the spring to adjust a baseline deformation of the spring, said force adjuster comprising a first screw (48) contained within said at least one of the first and second anchors;
**characterized in that** the first and second cylindrical anchors have respective first and second diameters, the first diameter being greater than the second diameter.

2. The device according to claim 1 , wherein the shock absorber comprises first and second shock absorbing elements, having different, respective responses to the force exerted on the cord.

3. The device according to claim 1, wherein the first and second anchors comprise respective collars (36, 38) at respective proximal ends of the anchors for engaging respective surfaces of the first and second bones, and wherein the device comprises a fastener configured to engage a distal end of the second anchor so as to secure the second anchor in the second bone.

4. The device according to claim 1 , wherein the first anchor comprises a collar for engaging a surface of the first bone when the first anchor is implanted inside the first bone, and wherein the collar contains one or more holes (27) that are configured to receive a suture for attachment to tissue in proximity to the first bone.

5. The device according to claim 1 , wherein the cord comprises multiple strands of a metal wire.

6. The device according to claim 1 , wherein the first and second anchors are sized and shaped for implantation inside the first and second metatarsal bones, respectively.

7. The device according to any of claims 1-6, further comprising an adjustment mechanism including a second screw (44) coaxial with the first screw and inside the same at least one of the first and second anchors as the first screw (48) to adjust a length of the cord extending between the first and second anchors.

8. A kit comprising the device according to claim 7, and comprising an adjustment tool (52), which comprises first and second coaxial drivers, which are configured to engage and turn the first and second screws, respectively.

9. The kit according to claim 8, wherein the adjustment tool comprises a tip (54), which engages a socket (50) in the at least one of the first and second anchors so as to prevent rotation of the cord as the first and second screws are turned.

10. The kit according to claim 9, wherein the tip is configured to be broken off the tool after adjustment of the force adjuster.

## Patentansprüche

1. Eine implantierbare Vorrichtung (20), die Folgendes aufweist:
a. eine erste zylindrische Verankerung (22), die so gestaltet ist, dass sie in einen ersten Knochen (24) implantiert werden kann;
b. eine zweite zylindrische Verankerung (26), die so gestaltet ist, dass sie in einen zweiten Knochen (28), neben dem ersten Knochen implantiert werden kann;
c. ein Kabel (30), das sich zwischen der ersten und der zweiten Verankerung erstreckt; und
d. ein Stoßdämpfer in mindestens entweder der ersten oder der zweiten Verankerung und gekoppelt mit dem Kabel, so dass er sich verformt als Reaktion auf eine auf das Kabel ausgeübte Kraft; dieser Stoßdämpfer weist eine Feder (42) und eine Kraft-Einstellvorrichtung auf, die so gestaltet ist, dass sie die Feder zusammendrückt, um eine Grundverformung der Feder einzustellen, diese Kraft-Einstellvorrichtung weist eine erste Schraube (48) auf, die in mindestens eine der besagten ersten oder zweiten Verankerung eingesetzt ist;
**dadurch gekennzeichnet, dass** die erste und zweite zylindrische Verankerung entsprechend einen ersten und zweiten Durchmesser haben, wobei der erste Durchmesser größer ist als der zweite Durchmesser.

2. Die Vorrichtung gemäß Anspruch 1, wobei der Stoßdämpfer ein erstes und zweites Stoßdämpfer-Element aufweist, mit jeweils verschiedenen Reaktionen auf die auf das Kabel ausgeübte Kraft.

3. Die Vorrichtung gemäß Anspruch 1, wobei die erste und zweite Verankerung jeweils Kränze (36, 38) an den jeweiligen proximalen Enden der Verankerungen aufweisen, um die jeweiligen Flächen des ersten und zweien Knochens zu halten, und wobei das Gerät ein Befestigungsteil aufweist, das so gestaltet ist, dass es ein distales Ende der zweiten Verankerung hält, um die zweite Verankerung im zweiten Knochen zu sichern.

4. Die Vorrichtung gemäß Anspruch 1, wobei die erste Verankerung einen Kranz aufweist, um eine Oberfläche des ersten Knochens zu halten, wenn die erste Verankerung in den ersten Knochen implantiert wurde, und wobei der Kranz ein oder mehrere Löcher (27) enthält, die so gestaltet sind, dass sie eine Naht für die Befestigung am Gewebe in der Nähe des ersten Knochens aufnehmen können.

5. Die Vorrichtung gemäß Anspruch 1, wobei das Kabel mehrere Stränge eines Metalldrahts aufweist.

6. Die Vorrichtung gemäß Anspruch 1, wobei die erste und zweite Verankerung so dimensioniert und geformt sind, dass sie in den ersten bzw. zweiten Mittelfußknochen implantiert werden können.

7. Die Vorrichtung gemäß eines der Ansprüche 1-6, die darüberhinaus einen Einstellmechanismus aufweist, der eine zweite Schraube (44) einschließt, die koaxial zur ersten Schraube und innerhalb der selben mindestens ersten oder zweiten Verankerung wie die erste Schraube (48) positioniert ist, um eine Länge des Kabels, das sich zwischen der ersten und zweiten Verankerung erstreckt, einzustellen.

8. Ein Kit, das die Vorrichtung gemäß Anspruch 7 aufweist, die wiederum ein Einstellwerkzeug (52) aufweist, das einen ersten und zweiten koaxialen Antrieb hat, die so gestaltet sind, dass sie die erste und zweite Schraube greifen und drehen können.

9. Das Kit gemäß Anspruch 8, wobei das Einstellwerkzeug eine Spitze (54) aufweist, die in eine Buchse (50) in mindestens entweder der ersten oder zweiten Verankerung einrastet, um zu verhindern, dass sich das Kabel dreht, wenn die erste und zweite Schraube gedreht werden.

10. Das Kit gemäß Anspruch 9, wobei die Spitze so gestaltet ist, dass sie vom Werkzeug abbricht, wenn die Kraft-Einstellvorrichtung eingestellt ist.

## Revendications

1. un dispositif implantable (20), consistant en :
a. une première ancre cylindrique (22), conçue pour être implantée à l'intérieur d'un premier os (24) ;
b. une seconde ancre cylindrique (26), conçue pour être implantée à l'intérieur d'un second os (28), adjacent au premier os ;
c. un cordon (30) s'étendant entre les première et seconde ancres ; et
d. un absorbeur de chocs contenu à l'intérieur d'au moins une des première et seconde ancres est couplé au cordon de manière à se déformer en réponse à une force exercée sur le cordon ; ledit absorbeur de chocs comprenant un ressort (42) et un ajusteur de force, qui est conçu pour comprimer le ressort pour ajuster une déformation de la base du ressort, ledit ajusteur de force comprenant une première vis (48) contenue à l'intérieur de ladite au moins une des première et seconde ancres ;
**caractérisé en ce que** les première et seconde ancres cylindriques présentent des premier et second diamètres respectifs, le premier diamètre étant plus grand que le second diamètre.

2. Le dispositif selon la revendication 1, dans lequel l'absorbeur de chocs comprend des premier et second éléments absorbants les chocs, présentant différentes réponses respectives à la force exercée sur le cordon.

3. Le dispositif selon la revendication 1, dans lequel les première et seconde ancres comprennent des colliers respectifs (36, 38) au niveau des extrémités proximales respectives des ancres destinées à venir en contact avec les surfaces respectives des premier et second os et dans lequel le dispositif comprend un élément de fixation conçu pour venir en contact avec une extrémité distale de la seconde ancre de manière à immobiliser la seconde ancre dans le second os.

4. Le dispositif selon la revendication 1, dans lequel la première ancre comprend un collier destiné à venir en contact avec une surface du premier os lorsque la première ancre est implantée à l'intérieur du premier os et dans lequel le collier contient un ou plusieurs trous (27) qui sont conçus pour recevoir une suture destinée à la fixation aux tissus à proximité du premier os.

5. Le dispositif selon la revendication 1, dans lequel le cordon comprend de multiples brins de fil métallique.

6. Le dispositif selon la revendication 1, dans lequel les première et seconde ancres sont dimensionnées et façonnées pour implantation à l'intérieur des premier et second os métatarsiens, respectivement.

7. L'appareil selon l'une quelconque des revendications 1 à 6, consistant en outre en un mécanisme d'ajustement, comprenant une seconde vis (44) coaxiale avec la première vis et à l'intérieur de celle-ci, au moins une des première et seconde ancres comme la première vis (48) pour ajuster la longueur du cordon s'étendant entre les première et seconde ancres.

8. Un kit comprenant le dispositif selon la revendication 7 et comprenant un outil d'ajustement (52), qui comprend des premier et second tournevis coaxiaux, qui sont conçus pour entrer en prise et faire tourner les première et seconde vis, respectivement.

9. Le kit selon la revendication 8, dans lequel l'outil d'ajustement comprend une pointe (54), qui entre en prise avec une douille (50) dans la ou les première et seconde ancres de manière à empêcher la rotation du cordon lorsque la première et la seconde vis sont tournées.

10. Le kit selon la revendication 9, dans lequel la pointe est conçue pour être séparée de l'outil après l'ajustement de l'ajusteur de force.
